# EUROPEAN PATENT SPECIFICATION

(11) **EP 2 163 630 B1**
(45) Date of publication and mention of the grant of the patent: **20.04.2016**
(21) Application number: 09169163.4
(22) Date of filing: 01.09.2009
(51) Int. Cl.: C12N 15/82, A01H 5/00

(54) **Method for controlling flowering time of plant**
Verfahren zur Steuerung der Blütezeit einer Pflanze
Procédé de contrôle du temps de floraison d'une plante

(30) Priority: 03.09.2008 JP 2008225711
(43) Date of publication of application: 17.03.2010
(73) Proprietor: SUMITOMO CHEMICAL CO., LTD., Tokyo 104-8260 (JP)
(72) Inventor: Saijo, Takanori, Osaka (JP); Nagasawa, Akitsu, Hyogo (JP)
(74) Representative: Vossius & Partner Patentanwälte Rechtsanwälte mbB

(56) References cited:
- WO-A2-2008/111661
- PENG XIANG-LEI ET AL: "Improvement of copper-inducible gene expression system for plant" ACTA BOTANICA SINICA, ZHIWU XUEBAO, KEXUE CHUBANSHE, CN, vol. 45, no. 11, 1 November 2003 (2003-11-01), pages 1307-1311, XP002491435 ISSN: 0577-7496
- GRANGER C L ET AL: "Characterization of the yeast copper-inducible promoter system in Arabidopsis thaliana" PLANT CELL REPORTS, vol. 20, no. 3, March 2001 (2001-03), pages 227-234, XP002559216 ISSN: 0721-7714
- SCHWECHHEIMER ET AL: "The activities of acidic and glutamine-rich transcriptional activation domains in plant cells: design of modular transcription factors for high-level expression" PLANT MOLECULAR BIOLOGY, SPRINGER, DORDRECHT, NL, vol. 36, no. 2, 1 January 1998 (1998-01-01), pages 195-204, XP002139229 ISSN: 0167-4412
- STORGAARD M ET AL: "Expression of a 434:VP16 chimeric activator leads to high-level activation of gene expression in stable transformants of Arabidopsis" TRANSGENIC RESEARCH, LONDON, GB, vol. 11, no. 2, 1 April 2002 (2002-04-01), pages 151-159, XP002348220 ISSN: 0962-8819
- PADIDAM MALLA: "Chemically regulated gene expression in plants." CURRENT OPINION IN PLANT BIOLOGY, vol. 6, no. 2, April 2003 (2003-04), pages 169-177, XP002559217 ISSN: 1369-5266
- KOBAYASHI, Y. ET AL.: "A pair of related genes with antagonistic roles in mediating flowering signals", SCIENCE, vol. 286, 3 December 1999 (1999-12-03), pages 1960-1962,

## Description

### BACKGROUND OF THE INVENTION

### Field of the Invention

The present invention relates to a method for controlling flowering time of plant using a chemical substance.

### Description of the Related Art

Flowering time of a plant is influenced by extrinsic factors such as photoperiod, temperature, and nutritional status, in addition to species-specific intrinsic factors. Accordingly, it is necessary to use special facilities such as a light irradiation device and a temperature control device for controlling environmental conditions in order to control flowering time of a plant, which raises cost problems. In addition, seeding time or a cultivation area is limited depending on a species, imposing a huge obstacle for improving productivity.

Recently, various genes involved in controlling flowering time in plant have been identified (Science 286, 1960-1962, 1999) and transgenic plants having altered flowering time have been produced by introducing those genes. In most of the plants, a foreign gene controlling flowering time is expressed under the control of a constitutive promoter which is constantly expressed.

In order to control flowering time of a transgenic plant by inducing expression of an introduced foreign gene which controls flowering time, an inducible system which has a high induction ratio and can be precisely regulated is required. That is, required is an inducible system which can decrease the expression of a foreign gene of interest to a low level under a non-inducible condition but can activate the expression of the foreign gene of interest under an inducible condition. A copper ion inducible system, which is one of the systems known as an inducible system, uses an inexpensive inducing agent and can be used in the field (Proc Natl Acad Sci 90, 4567-45712, 1993). However, the conventionally known copper ion inducible system has a problem that, expression of a foreign gene under non-inducing condition cannot be inhibited to a sufficient low level, expression of a foreign gene under inducing condition cannot be activated to a sufficient high level, and a copper ion as an inducing agent should be treated at a high concentration for a long period of time, resulting in significant toxic effects (Current Opin Plant Biol 6, 169-177, 2003). Peng (Acta Botanica Sinica 45, 1307-1310, 2003) and Granger (Plant Cell Reports 20, 227 - 234, 2001) disclose copper-inducible systems in plants. Schwechheimer (Plant Mol. Biol. 36, 195 - 198, 1998) discloses transcriptional activation domains.

### SUMMARY OF THE INVENTION

It is an object of the present invention to provide a method of controlling flowering time of a transgenic plant in a copper ion-dependent manner, by inducing the expression of a foreign gene which controls flowering time using an inducible system that can induce the expression of the foreign gene at a high induction ratio.

The present invention provides:
1. a method for controlling flowering time of a plant, which comprises bringing a plant into contact with copper ions, wherein the plant has been transformed with the following DNA constructs (i) and (ii):
   (i) a first DNA construct comprising a nucleotide sequence encoding a chimeric transcription factor, wherein the chimeric transcription factor comprises the following a) and b) as operably linked elements:
      a) a DNA-binding domain and a transcriptional activation domain of a first transcription factor that is capable of being activated by copper ion, and
      b) a transcriptional activation domain of at least one second transcription factor that is different from the first transcription factor; and
   (ii) a second DNA construct encoding a heterologous gene which controls flowering time of a plant, wherein the gene is under the control of a heterologous promoter capable of being stimulated by the chimeric transcription factor of (i) in the presence of copper ion
      (hereinafter, sometimes, referred to as the present controlling method);
2. the method according to the item 1, wherein the first DNA construct further comprises a terminator which is capable of functioning in plant cells and is operably linked to the nucleotide sequence encoding the chimeric transcription factor, and wherein the second DNA construct further comprises a terminator which is capable of functioning in plant cells and is operably linked to the coding sequence in the heterologous gene.
3. the method according to the item 1 or 2, wherein the first transcription factor is selected from the group consisting of:
   (1) a eukaryote transcription factor which is capable of being activated by copper ion,
   (2) a yeast transcription factor which is capable of being activated by copper ion, and
   (3) a transcription factor derived from the yeast ACE1 which is capable of being activated by copper ion;
4. the method according to any of the items 1 to 3, wherein the second transcription factor is selected from the group consisting of:
   (1) a transcription factor of a virus,
   (2) a transcription factor of a virus belonging to Simplex virus genus,
   (3) a transcription factor of Herpes simplex virus, and
   (4) the VP16 transcription factor of Herpes simplex virus;
5. the method according to any of the items 1 to 4,
   wherein the heterologous gene which controls flowering time of a plant is selected from the group consisting of:
   (1) a plant gene controlling flowering time,
   (2) an angiosperm gene controlling flowering time,
   (3) a dicotyledonous gene controlling flowering time,
   (4) a Cruciferous gene controlling flowering time,
   (5) an *Arabidopsis thaliana* gene controlling flowering time, and
   (6) a flowering-time controlling gene derived from the *Arabidopsis thaliana* FT gene;
6. the method according to any of the items 1 to 5,
   wherein the first DNA construct further comprises a promoter which is capable of functioning in plant cells and is operably linked to the nucleotide sequence encoding the chimeric transcription factor.
   The present disclosure provides
7. a set of the following DNA constructs (i) and (ii):
   (i) a first DNA construct comprising a nucleotide sequence encoding a chimeric transcription factor, wherein the chimeric transcription factor comprises the following a) and b) as operably linked elements:
      a) a DNA-binding domain of a first transcription factor that is capable of being activated by copper ion, and
      b) a transcriptional activation domain of at least one second transcription factor that is different from the first transcription factor; and
   (ii) a second DNA construct encoding a heterologous gene which controls flowering time of a plant, wherein the gene is under the control of a heterologous promoter capable of being stimulated by the chimeric transcription factor of (i) in the presence of copper ion
      (hereinafter, sometimes, referred to as the present DNA constructs);
8. the set of the DNA constructs according to the item 7, wherein the chimeric transcription factor further comprises as the operably linked elements a transcriptional activation domain of the first transcription factor;
9. the set of the DNA constructs according to the item 7 or 8, wherein the first transcription factor is selected from the group consisting of:
   (1) a eukaryote transcription factor which is capable of being activated by copper ion,
   (2) a yeast transcription factor which is capable of being activated by copper ion, and
   (3) a transcription factor derived from the yeast ACE1 which is capable of being activated by copper ion;
10. the set of the DNA constructs according to any of the items 7 to 9, wherein the second transcription factor is selected from the group consisting of:
   (1) a transcription factor of a virus,
   (2) a transcription factor of a virus belonging to Simplex virus genus,
   (3) a transcription factor of Herpes simplex virus, and
   (4) the VP16 transcription factor of Herpes simplex virus;
11. the set of the DNA constructs according to any of the items 7 to 10, wherein the heterologous gene which controls flowering time of a plant is selected from the group consisting of:
   (1) a plant gene controlling flowering time,
   (2) an angiosperm gene controlling flowering time,
   (3) a dicotyledonous gene controlling flowering time,
   (4) a Cruciferous gene controlling flowering time,
   (5) an *Arabidopsis thaliana* gene controlling flowering t ime, and
   (6) a flowering-time controlling gene derived from the *Arabidopsis thaliana* FT gene;
12. the set of the DNA constructs according to any of the items 7 to 11, wherein the first DNA construct further comprises a promoter which is capable of functioning in plant cells and is operably linked to the nucleotide sequence encoding the chimeric transcription factor;
13. a plant transformed with the set of DNA constructs of any of the items 7 to 11 (hereinafter, sometimes, referred to as the present transformed plant); and
14. a method of producing a transgenic plant with a copper-inducible control of flowering time comprising:
   introducing the set of DNA constructs of any of the items 7 to 11 into a plant cell,
   regenerating a plant from the plant cell,
   selecting a plant comprising the DNA constructs, and
   growing the plant under conditions that allow copper-inducible control of flowering time.

According to the present invention, a method for controlling flowering time of a transgenic plant in a copper ion-inducible manner is provided, wherein a heterologous gene which controls flowering time has been introduced to the plant. Thus, regardless of an environment, seeding time or cultivation area, flowering can be induced at any desired time so that systematic harvest and shipping based on precisely estimated needs, efficient production of F₁ hybrid seeds, prevention of gene leakage from a genetically-engineered crop, efficient production of leaf vegetables or root vegetables, improvement of productivity for flowers and ornamental plants, efficient breed improvement based on reduced period between generations, increased harvest amount, and the like may be expected to be achieved.

### BRIEF DESCRIPTION OF THE DRAWINGS

Fig. 1 is a schematic view illustrating the structures of T-DNA regions of copper ion-inducible sGFP gene expression vectors and a copper ion-inducible FT gene expression vector. The symbol "*" in Fig. 1 shows a mutation-introduced region of the as-1 site.
Fig. 2 shows difference in flowering time of the recombinant *Arabidopsis thaliana* in which the copper ion-inducible FT gene expression vector has been introduced.

### DETAILED DESCRIPTION OF THE PREFERRED EMBODIMENTS

Herein below, the present invention will be described in more detail.

The present DNA constructs can be used for inducing in chemical substance-dependent manner expression of a heterologous gene which controls flowering time of a plant.

The present DNA constructs can be constructably prepared so as to comprise:
(i) a first DNA construct comprising a nucleotide sequence encoding a chimeric transcription factor (hereinafter, sometimes, referred to as the present chimeric transcription factor), wherein the chimeric transcription factor comprises the following a) and b) as operably linked elements:
   a) a DNA-binding domain of a first transcription factor that is capable of being activated by copper ion, and
   b) a transcriptional activation domain of at least one second transcription factor that is different from the first transcription factor; and
(ii) a second DNA construct encoding a heterologous gene which controls flowering time of a plant (hereinafter, sometimes, referred to as the present flowering-time control gene), wherein the gene is under the control of a heterologous promoter capable of being stimulated by the chimeric transcription factor of (i) in the presence of copper ion.

An "expression cassette" described below indicates a DNA construct which can express a structural gene, such as the present flowering-time control gene, comprised in the construct in a host cell. Examples of the expression cassette include a DNA construct in which a promoter and a terminator are operably linked respectively to the upstream and the downstream of a structural gene.

The present chimeric transcription factor can be activated by copper ions and can induce the transcription of the present flowering-time control gene. In the absence of copper ions, the present chimeric transcription factor is in an inactive form, and therefore transcription of the present flowering-time control gene is not substantially induced. In the presence of copper ions, the present chimeric transcription factor is converted into an active form, and therefore can induce the transcription of the present flowering-time control gene.

A transcription factor generally contains a DNA binding domain and a transcriptional activation domain. The transcriptional activation domain has a function to recruit mediators for a RNA polymerase complex in a host plant cell and activate the transcription of a target gene.

In the present invention, the present chimeric transcription factor comprises as operably linked elements
a) a DNA-binding domain of a first transcription factor that is capable of being activated by copper ion and
b) a transcriptional activation domain of at least one second transcription factor that is different from the first transcription factor.

The present chimeric transcription factor may further comprise as the operably linked elements a transcriptional activation domain of the first transcription factor. That is, examples of the present chimeric transcription factor also include a chimeric transcription factor comprises as operably linked elements
a-1) a DNA-binding domain and a transcriptional activation domain of a first transcription factor that is capable of being activated by copper ion, and
b) a transcriptional activation domain of at least one second transcription factor that is different from the first transcription factor.

The present chimeric transcription factor can induce the expression of the present flowering-time control gene at a high induction ratio because of its structural feature that it comprises a transcriptional activation domain of at least one second transcription factor that is different from the first transcription factor. In order to control the flowering time of the present transformed plant, higher ratio for inducing the expression of the present flowering-time control gene is preferable.

Examples of the "first transcription factor" include a transcription factor selected from the group consisting of:
(1) a eukaryote transcription factor which is capable of being activated by copper ion,
(2) a yeast transcription factor which is capable of being activated by copper ion, and
(3) a transcription factor derived from the yeast ACE1 (activating copper-metallothionein expression 1) which is capable of being activated by copper ion.

Examples of the first transcription factor also include AMT1 of *Candida glabrata* (J Biol Chem, 268, 12512-12518, 1993), CRF1 of *Yarrowia lipolytica* (J Biol Chem, 277,37359-37368, 2002), a transcription factor which can induce the expression of the corn SOD gene cluster in a copper ion-dependent manner (Biochemistry, 42,1508-1516,2003).

The "second transcription factor" is a transcription factor different from the first transcription factor as described above and a transcriptional activation domain of the second transcription factor allows the present chimeric transcription factor to induce the expression of the present flowering-time control gene at a high induction ratio. Examples of the "second transcription factor" include a transcription factor selected from the group consisting of:
(1) a transcription factor of a virus,
(2) a transcription factor of a virus belonging to Simplex virus genus,
(3) a transcription factor of Herpes simplex virus, and
(4) the VP16 transcription factor of Herpes simplex virus (Genes Dev 2(6), 718-29, 1998).

Examples of the second transcription factor also include GAL4 transcription factor (Cell 51(1), 121-126, 1987), and an artificially synthesized peptide AH (Chem Biol 8(6), 583-592, 2001) having an ability of activating transcription.

A "heterologous gene which controls flowering time of a plant" utilized (i.e., the present flowering-time control gene) can be a gene which promotes flowering or a gene which delays flowering. Preferably, it is appropriately selected in accordance with the intended use. That is, the term "controls flowering time" for the present contlloring method means either to promote flowering or to delay flowering, depending on the nature of the present flowering-time control gene.

Examples of a gene which promotes flowering include FT, CO, GI, SOC1, AGL24, LD, FCA, FUL, LFY, API, TSF, and FD genes and also include any genes which can promote flowering. In addition, genes which promote flowering include dominant-negative mutants, antisense genes and RNAi-inducing genes for genes which delay flowering as described below. It is preferable that genes which integrate pathways each relating to determination of flowering time be used, the pathways including photoperiod-dependent promotion pathway, autonomous promotion pathway, vernalization-dependent promotion pathway and gibberellin-dependent promotion pathway, and the genes including FT, SOCI and LFY genes. It is more preferable that FT gene, which may be involved in the production of florigen, and homologous genes thereof be used.

Examples of a gene which delays flowering include FLC, FRI, PIE1, TFL2, VIP1-7, LHY, SVP, SPA, and CDF1 genes and also include any genes which can delay flowering. In addition, genes which delay flowering include dominant-negative mutants, antisense genes and RNAi-inducing genes for genes which promote flowering as described above. It is preferable that dominant-negative mutants, antisense genes or RNAi-inducing gene for genes which integrate pathways each relating to determination of flowering time be used, the pathways including photoperiod-dependent promotion pathway, autonomous promotion pathway, vernalization-dependent promotion pathway and gibberellin-dependent promotion pathway, and the genes including FT, SOCI and LFY genes. It is more preferable that dominant-negative mutants, antisense genes or RNAi-inducing gene for FT gene and homologous genes thereof be used.

Specifically, the FT gene of *Arabidopsis thaliana* (Science 286, 1960-1962, 1999) has been known and its homologous gene was identified as Hd3a gene in rice (Science 316, 1033-1036, 2007). Genes homologous to *Arabidopsis* FT gene have also been identified from morning glory, orange, poplar, barley, grape, apple and the like. In addition to the *Arabidopsis* FT gene, many genes which control flowering time have been identified from various plant species. Some of the genes controlling flowering time can function across plant species. While it is preferable that a flowering-time control gene originating from a host plant species into which the gene will be introduced, a flowering-time control gene originating from other plant species may also be used, without limitation, if the gene can control flowering time in a host plant species of interest.

More detailed information regarding the above-described genes which controls flowering time is given in some review articles (for example: Genes Dev 21,2371-84, 2007; and Bioessays 26, 363-373, 2004).

Examples of the present flowering-time control gene include a heterologous gene selected from the group consisting of:
(1) a plant gene controlling flowering time,
(2) an angiosperm gene controlling flowering time,
(3) a dicotyledonous gene controlling flowering time,
(4) a Cruciferous gene controlling flowering time,
(5) an *Arabidopsis thaliana* gene controlling flowering time, and
(6) a flowering-time controlling gene derived from the *Arabidopsis thaliana* FT gene.

FT gene is an abbreviation of a Flowering Locus T gene, and means a gene encoding a factor that positively functions in flowering-time control. Expression of FT gene increases at a sieve part of fibrovascular bundle in response to length of day, and then the expressed FT moves to a top of a stem and interacts with a bZIP type transcription factor called FD expressed on a top of the stem, thereby inducing flowering (Genes Dev 21, 2371-2384, 2007).

In the present invention, the present flowering-time control gene is under the control of a heterologous promoter capable of being stimulated by the present chimeric transcription factor in the presence of copper ion. Specifically, in the present flowering-time control gene, a heterologous promoter sequence capable of being stimulated by the present chimeric transcription factor in the presence of copper ion (hereinafter, sometimes, referred to as the present promoter sequence) is operably linked in sense or antisense orientation to a coding sequence of the gene. Any sequence can be used as the present promoter sequence as far as the present flowering-time control gene can be expressed in the host cells under inducible conditions. Preferably, for example, there can be mentioned a promoter sequence containing MRE sequences of yeast (Proc Natl Acad Sci 90, 4567-4571, 1993). Specifically, there is mentioned a promoter sequence in which MRE sequences of yeast are repeatedly located upstream of a TATA sequence of a commonly used promoter. In the present flowering-time control gene, no 5'-untranslated sequence of a foreign gene is required to be inserted between the promoter sequence and the coding sequence. For example, the promoter sequence and the coding sequence may be substantially directly linked.

Preferred examples of the present DNA constructs include a DNA construct in which the first DNA construct further comprises a promoter which is capable of functioning in plant cells and is operably linked to the nucleotide sequence encoding the chimeric transcription factor. Examples of the promoter include a commonly used promoter described below.

The commonly used promoter described above can be a constitutive promoter, a tissue-specific promoter or an inducible promoter that induce transcription by certain stimulation. Preferably, it is appropriately selected in accordance with the intended use.

Examples of the constitutive promoter include CaMV 35S promoter, PG10-90 (US Patent No.6187996), ubiquitin promoter (International Publication No.01/094394), actin promoter (International Publication No.00/070067) and the like. Further, examples of the tissue-specific promoter include soybean seed glycinin promoter (EP Publication No.0571741), prolamine promoter (International Publication No.2004/056993), kidney bean seed phaseolin promoter (International Publication No.91/013993), rape seed napin promoter (International Publication No. 91/013972), *Arabidopsis thaliana* Sultr2;2 promoter (Plant J 23,171-82, 2000), and Agrobacterium rolC promoter (Physiol 115,1599-1607, 1997).

Preferred examples of the present DNA constructs also include a gene DNA construct in which the first DNA construct further comprises a terminator which is capable of functioning in plant cells and is operably linked to the nucleotide sequence encoding the chimeric transcription factor. Examples of the terminator include NOS terminator, CR16 terminator (US Patent No.7202083), and soybean seed glycinin terminator (EP Publication No.0571741).

The present DNA constructs can be constructably prepared so as to contain the respective elements (i) and (ii) described above. That is, it can be constructed so as to contain the respective elements (i) and (ii) on a single vector, or it can be constructed by using two vectors so as to contain the element (i) on one vector and to contain the element (ii) on the other vector.

Such vector can be constructed by using a conventional genetic engineering method. In addition, when the present DNA constructs are constructed by using two vectors, it is possible that the above (i) is constructed as a first vector and the above (ii) is constructed as a second vector, for example.

The present DNA constructs are introduced, for example, into a host plant and induce expression of the present DNA constructs. The introduction of the present DNA constructs into a host plant can be carried out according to a conventional genetic engineering technique, according to a method applicable to each host plant.

Specifically, the present DNA constructs can be introduced by using a single vector that is constructed so as to contain the respective elements (i) and (ii) described above, or those can be introduced by mixing the first vector that is constructed so as to contain the above (i) with the second vector that is constructed so as to contain the above (ii). In addition, to a host plant in which part of the present DNA constructs has been introduced by using any of the first vector or the second vector, the rest of the present DNA constructs can be further introduced by using the remaining vector. In addition, the present DNA constructs can be introduced into a host plant by crossbreeding between a plant in which part of the present DNA constructs has been introduced by using the first vector and a plant in which the other part of the present DNA constructs has been introduced by using the second vector. It is also possible that multiple kinds of the second vector are constructed using multiple kinds of flowering-time controlling genes and obtained DNA constructs are introduced thereto to simultaneously induce the expression of multiple kinds of genes which controls flowering time.

As the method of introducing the present DNA constructs, there can be used various known methods such as Agrobacterium method, particle gun method, electroporation method, calcium phosphate method, and virus vector method.

Examples of the host plant include species important for agriculture and gardening, or useful in genetics such as genome analysis. Further, the host plant includes arbitrary plant species. Examples thereof include soybean, pea, kidney bean, alfalfa, Lotus japonicus, clover, peanut, sweet pea, walnut, tea, cotton, pepper, cucumber, water melon, pumpkin, squash, melon, radish, rapeseed, canolla, beet, lettuce, cabbage, broccoli, cauliflower, Arabidopsis, tobacco, eggplant, potato, sweet potato, taro, artichoke, tomato, spinach, asparagus, carrot, sesame, endive, chrysanthemum, geranium, antirrhinum, carnation, pink, sweet oleander, Bouvardia, Gypsophilla, gerbera, Russell prairie gentian, tulip, Mathiola incana, Limonium, cyclamen, Saxifraga stolonifera, swamp chrysanthemum, violet, rose, cherry, apple, strawberry, Japanese apricot, orange, Japanese quince, azalea, Barbados nut, gentian, cosmos, morning-glory, sunflower, ginkgo, Japanese cedar, Japanese cypress, poplar, pine, Sequoia, oak, water lily, Eucommia, beech, rice, wheat, barley, rye, oat, corn, maize, green onion, garlic, lily, Tiger lily, orchid, gladiolus and pineapple.

In the present controlling method, the present transformed plant is brought into contact with copper ions. Examples of the contact method include a method of adding copper ions to a medium or nutrient solution when the present transformed plant is cultivated in the medium or hydroponically cultivated (specifically, the concentration of copper ions is 1 µM to 24 mM, for example). Further, when the present transformed plant is cultivated in soil, a method in which copper ions can be applied to the soil or to the stems and leaves of the present transformed plant (specifically, the amount of copper ions is from 1 nmol/plant to 2.4 mmol/plant, and the concentration of copper ions is 1 µM to 24 mM, for example) can be mentioned. Depending on types of host plants and specific condition of use, an appropriate method is preferably selected. In the present contlloring method, it is required to penetrate copper ion into plant cells wherein induced expression of a flowering-time controlling gene is expected. For the purpose of this penetration, for example, a preparation of a complex of copper ion to be applied can be used. Alternatively, a copper agent for agriculture applications such as Bordeaux (Sumitomo Chemical Co., Ltd.), G-fine (Yashima Chemical Industry Co., Ltd.), Tomono Z-Bordeaux (Tomono Agrica Co., Ltd.), Ridomilpulus (Nihon Nohyaku Co., Ltd.), Highcopper (Sumitomo Chemical Co., Ltd.), CUPRAVIT forte (Bayer CropScience Corporation), Kocide Bordeaux (DuPont Corporation), Kinondo (Agro Kanesho Co., Ltd.) or Yonepon (Yonezawa Chemical Co., Ltd.); or a copper agent to be used as food additives such as copper gluconate (Wako Pure Chemical Industries, Ltd.) can be employed by adjusting to a desired concentration. Further, a spreading agent can be mixed when a preparation or an agent is applied.

The contact of the present transformed plant with copper ion described above may be carried out by applying a mixture of copper ions and fertilizer or a conventional agricultural pest control agent such as insecticide, fungicide, herbicide and plant growth regulator. The contact of the present transformed plant with copper ion may also be carried out when the present transformed plants are irrigated.

The DNA constructs used for the present controlling method is, for example, the present DNA constructs as described above. Further, the transcription factor used for the present controlling method is the present chimeric transcription factor as described above, and it is activated by copper ions. Further, the gene which controls plant flowering time that is used for the present controlling method is the present flowering-time control gene as described above, and its transcription is induced by the present chimeric transcription factor that is activated by copper ions. In addition, the transcriptional activation domain that is used for the present controlling method is the transcriptional activation domain as described above, and it is contained in the present chimeric transcription factor that can be activated by copper ions.

The present transformed plant is a transgenic plant which is obtained by introducing the present DNA constructs thereto, as described above.

When the present flowering-time control gene is a gene for promoting flowering, by introducing the present DNA constructs to a plant having a difficulty in flowering or no flowering due to a mutation introduced to a flowering promotion gene, RNAi, or overexpression of a gene which delays flowering, etc., more precise control of flowering time can be achieved. On the other hand, when the present flowering-time control gene is a gene for delaying flowering, by introducing the present DNA constructs to a plant having precocious flowering due to a mutation introduced to a flowering delay gene, RNAi, or overexpression of a gene which promotes flowering, etc., more precise control of flowering time can be achieved.

The present invention is not limited to each embodiment described above. Within the scope described in the claims, various modifications can be made, and an embodiment which is obtained by appropriate combination of technical means that is separately described in different embodiments is also within the technical scope of the present invention.

### EXAMPLES

Herein below, the present invention will be described in more detail by way of examples. However, the present invention is not limited thereto.

### Example 1 (Construction of an introduction vector)

### (1) Construction of transcription factor gene expression cassettes

A genomic DNA was extracted from budding yeast (Saccharomyces cerevisiae strain AH22) cultured with shaking at 30°C in a YPD medium (1% yeast extract, 2% polypeptone, 2% glucose) for 2 days by using a genome DNA extraction kit "Gentorukun" (Takara Bio, Inc.). By using the extracted genome DNA as a template, an ACE1 transcription factor gene was amplified by PCR using two kinds of specific primers (ACE1-1F, ACE1-1RC). The amplified ACE1 transcription factor gene was cloned into pBI221 (Clontech) by replacing a GUS gene of pBI221 by the amplified ACE1 transcription factor gene to prepare p35S-ACE1-NOS. Next, a NOS terminator contained in the p35S-ACE1-NOS was replaced by a CR16 terminator (US Patent No.7202083) to prepare p35S-ACE1-CR.

Seeds of recombinant *Arabidopsis thaliana* (No.N70016), which had been purchased from NASC (Nottingham Arabidopsis Stock Centre), were plated on a modified MS agar medium (MS inorganic salts, B5 vitamin, 2% sucrose, 0.8% agar). From the true leaves of the recombinant *Arabidopsis thaliana* which had been grown for three weeks at 23°C, genomic DNA was extracted by using a kit for extracting plant genomic DNA, "DNeasy Plant mini kit (QIAGEN)." Using the extracted genomic DNA as a template, PCR was carried out by using two kinds of specific primers (VP16-1F, VP16-1RC) to amplify the gene for transcriptional activation domain (VP16AD) of transcription factor VP16 of a Herpes simplex virus. The amplified VP16AD gene was subjected to TA cloning into pCR2.1 (Invitrogen) to prepare pCR2.1-VP16AD. Using the pCR2.1-VP16AD as a template, PCR was carried out by using two specific kinds of primers (VP16-2F, VP16-2RC) and mutation was introduced into SacI site in the VP16AD gene to prepare pCR2.1-VP16AD(dSacI). Then, using the pCR2.1-VP16AD(dSacI) as a template, PCR was carried out by using two kinds of specific primers (VP16-3F, VP16-3RC) to add an XhoI site to the 5'-terminal, and SacI site to the 3'-terminal of the VP16AD(dSacI) gene. Furthermore, using the pCR2.1-VP16AD(dSacI) as a template, PCR was carried out by using two kinds of specific primers (VP16-4F, VF16-3RC) to add an BglII site to the 5'-terminal, and SacI site to the 3'-terminal of the VP16AD(dSacI) gene.

Using p35S-ACE1-CR as a template, PCR was carried out by using two kinds of specific primers (ACE1-1F, ACE1-2RC) to remove the termination codon of the ACE1 transcription factor gene and to add XhoI site thereto. To the downstream of the ACE1 transcription factor gene to which XhoI site had been added to the 3'-terminal, VP16AD (dSacI) gene in which XhoI site had been added to the 5'-terminal was ligated in frame. As a result, p35S-ACE1/VP16AD-CR was prepared. Further, p35S-ACE1-CR was treated with the restriction enzymes BglII and SacI to cut out ACE1AD gene. To the downstream of the ACE1DBD transcription factor gene that lacks ACE1AD gene, VP16AD(dSacI) gene in which BglII site had been added to the 5'-terminal was ligated in frame to replace the ACE1AD gene by the VP16AD(dSacI) gene. As a result, p35S-ACE1DBD/VP16AD-CR was prepared.
ACZ1-1F : 5'-atggatccatggtcgtaattaacggg-3' (SEQ ID NO:1)
ACE1-1RC : 5'-tggagctcttattgtgaatgtgagttatg-3' (SEQ ID NO:2)
ACE1-2RC : 5'-aactcgagttgtgaatgtgagttatgcg-3'(SEQ ID NO:3)
VP16-1F: 5'-acggctccaccgaccgacgtc-3' (SEQ ID NO:4)
VF16-2RC : 5'-ctacccaccgtactcgtcaattc-3' (SEQ ID NO:5)
VP1C-2F: 5'-ggacgaactccacttagacgg-3' (SEQ ID NO: 6)
VP16-2RC : 5'-ccgtctragtggaattcgtcc-3' (SEQ ID NO:7)
VP16-3F : 5'-tactcgagtcaacggctccaccgaccgacgt-3' (SEQ ID NO:8)
VP16-3RC : 5'-aagagctcttacccaccgtactcgtcaattccaag-3' (SEQ ID NO:9)
VF16-4F : 5'-taagatctatcaacggctccaccgaccgacgt-3' (SEQ ID NO:10)

### (2) Construction of an expression cassette for sGFP gene

An sGFP gene was cut out from the plasmid CaMV35S-sGFP(S65T)-NOS3'("Experimental protocol for observation of a plant cell", edited by Fukuda Hiroo et al., 1997, Shujunsha, ISBN 4-87962-170-6), and cloned into pBI221, by using an adaptors NS-1F and NS-1RC and replacing a GUS gene of pBI221 by the sGFP gene to prepare p35S-sGFP. A region from -830bp to -91bp of CaMV35S promoter that is contained in p35S-sGFP was replaced by synthetic oligonucleotides MRE-1F and MRE-1RC to prepare pMRE/35S(-90)-sGFP.

The pMRE/35S(-90)-sGFP was treated with the restriction enzyme EcoRV, and then dephosphorylated by using "Calf intestine Alkaline Phosphatase" (TAKARA BIO). Then, the blunt-ended and phosphorylated synthetic oligonucleotides MRE-1F and MRE-1RC were inserted thereto by using "Blunting Kination Ligation kit" (TAKARA BIO) to arrange MRE sequences repeatedly twice in the forward direction. The portion in which the MRE sequence is repeated twice was cut out, and then was blunt-ended according to the method same as described above, and inserted again into EcoRV site. As a result, pMRE4/35S(-90)-sGFP in which MRE sequence is repeatedly aligned four times in the forward direction was prepared.

In addition, using pBI221 as a template, PCR was carried out by using two kinds of specific primers (46bp-1F, 46bp-1RC) to amplify a DNA fragment containing a region from ∼46bp to - 1bp of CaMV35S promoter. The amplified DNA fragment was substituted for the region encompassing from -90bp to -1b of CaMV35 promoter, which is located downstream of the MRE sequence that had been repeatedly aligned four times in the forward direction in the pMRE4/35S(-90)-sGFP, to give pMRE4/35S(-46)-sGFP.

Meanwhile, a replacement was carried out with a sequence in which a mutation had been introduced to an as-1 site present in the region encompassing from -90bp to -1bp of CaMV35S promoter (Benfey PN & Chua NH, 1990, Science 250, 959-966). First, genomic DNA was extracted from *Arabidopsis thaliana* (No. N70016) purchased from NASC. Using the extracted genomic DNA as a template, PCR was carried out by using two kinds of specific primers (90m-1F, 90m-1RC). Then, 35S(-90m) sequence containing a mutation at an as-1 site was subjected to TA cloning into pCR2.1 (Invitrogen) to prepare pCR2.1-35S(-90m). Using pCR2.1-35S(-90m) as a template, PCR was carried out by using two kinds of specific primers (90m-2F, 90m-2RC) to add EcoRV site to the 5'-terminal and XbaI site to the 3'-terminal. The resulting DNA fragment was substituted for the region encompassing from -90bp to -1bp of CaMV35S promoter; which is located downstream of the MRE sequence that had been repeatedly aligned four times in the forward direction in the pMRE4/35S(-90)-sGFP, to give pMRE4/35S(-90m)-sGFP.

Each of the pMRE4/35S(-46)-sGFP and the pMRE4/35S(-90m)-sGFP was treated with the restriction enzyme HindIII, blunt-ended and phosphorylated by using "Blunting Kination Ligation kit", and then synthetic oligonucleotides KXS-1F and KXS-1RC were inserted thereto to prepare pKXS-MRE4/35S(-46)-sGFP or pKXS-MRE4/35S(-90m)-sGFP.
NS-1F : 5'-ggccgcgagctcagt-3' (SEQ ID NO:11)
NS-1RC : 5'-gactgagctcgc-3' (SEQ ID NO:12)
MRE -1F : 5' - agcttagcgatgcgtcttttccgctgaaccgttccagcaaaaaagactagat-3' (SEQ ID NO:13)
MRE-1RC : 5'-atctagtcttttttgctggaacggttcagcggaaaagacgcatcgcta-3' (SEQ ID NO:14)
46bp-1F : 5'-tagatatcgcaagacccttcctctatataagg-3' (SEQ ID NO:15)
46bp-1RC : 5'-atcctctagagtcccccgtgttc-3' (SEQ ID NO:16)
90m-1F : 5'-gctatgaccatgattacgccaagcttg-3' (SEQ ID NO:17)
90m-1RC : 5'-cattgttatatctccttggatccgtcg-3' (SEQ ID NO:18)
90m-2F : 5'-tagatatctccacgtccataagggac-3' (SEQ ID NO:19)
90m-2RC: 5'-aatctagactgcaggtcgtcctctcca-3' (SEQ ID NO:20)
KXS-1F : 5'-ggtacctcgagtcgac-3' (SEQ ID NO:21)
KXS-1RC : 5'-gtcgactcgaggtacc-3' (SEQ ID NO:22)

### (3) Construction of an expression cassette for FT gene

Seeds of *Arabidopsis thaliana* (Arabidopsis thaliana ecotype Columbia) were plated on a modified MS agar medium (MS inorganic salts, B5 vitamin, 2% sucrose, 0.8% agar), and grown at 23°C for three weeks. The obtained plant was then transplanted to a pot already filled with culture soil and cultivated in an artificial weather chamber. Two weeks after the transplantation, total RNA was extracted from the flower bud and true leaf of the plant by using a plant RNA extracting kit "RNeasy Plant Mini Kit" (QIAGEN). With the extracted total RNA, cDNA was synthesized by using a cDNA synthesis kit "ReverTra Ace" (TOYOBO). Using the synthesized cDNA as a template, PCR was carried out by using two kinds of specific primers (FT-1F, FT-1RC) to amplify FT gene (GenBank Accession Number AB027504). Then, the amplified FT gene was substituted for GUS gene of pBI221 (Clontech). Using thus obtained plasmid as a template, fusion PCR was carried out by using six kinds of specific primers (FT-2F, FT-2RC, FT-3F, FT-3RC, FT-4F, FT-1RC). As a result, a mutation without causing amino acid substitution was introduced at the BamHI site and EcoRI site present in the FT gene, and also an XbaI site at the 5'-terminal was changed to a BamHI site. The resulting modified FT gene was substituted for sGFP gene of pKXS-MRE4/35S(-90m)-sGFP to prepare pKXS-MRE4/35S(-90m)-FT.
FT-1F : 5'-taatctagaatgtctataaatataagagaccctc-3' (SEQ ID NO:23)
FT-1RC : 5'-atagagctcctaaagtcttcttcctccg-3' (SEQ ID NO:24)
FT-2F : 5'-taaggatccatgtctataaatataagagaccctc-3' (SEQ ID NO:25)
FT-2RC 5'-gaacatctggatcgaccataaccaaagta-3' (SEQ ID NO:26)
FT-3F : 5'-tactttggttatggtcgatccagatgttc-3' (SEQ ID NO:27)
FT-3RC : 5'-gacacgatgaatacctgcagtggga-3' (SEQ ID NO:28)
FT-4F : 5'-tcccactgcaggtattcatcgtgtc-3' (SEQ ID NO:29)

### (4) Linking an expression cassette for transcription factor gene to an expression cassette for sGFP gene or to an expression cassette for FT gene

Plasmids p35S-ACE-CR, p35S-ACE1DBD/VP16AD-CR, and p35S-ACE1/VP16AD-CR, containing expression cassettes for transcription factor gene, were treated with the restriction enzymes HindIII and EcoRI to cut out the expression cassettes for transcription factor gene. Plasmids pKXS-MRE4/35S(-46)-sGFP and pKXS-MRE4/35S(-90m)-sGFP containing expression cassettes for sGFP gene and pKXS-MRE4/35S(-90m)-FT containing an expression cassette for FT gene were treated with the restriction enzymes KpnI and EcoRI to cut out the expression cassettes for sGFP gene and the expression cassette for FT gene, respectively.

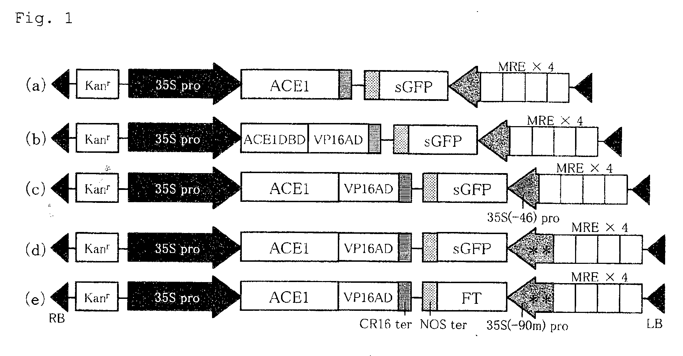

An expression cassette for GUS gene contained in pBI121 (Clontech) was replaced by synthetic oligonucleotides HEK-1F and HEK-1RC to prepare pBI121-HEK. The pBI121-HEK was then treated with the restriction enzymes HindIII and KpnI. To the pBI121-HEK which had been treated with HindIII and KpnI, each one of the expression cassettes for transcription factor gene, and each one of the expression cassettes for sGFP gene or the expression cassette for FT gene, which had been cut out as described above, were ligated to obtain binary vectors in each of which the expression cassette for transcription factor gene and the expression cassette for SGFP gene or the expression cassette for FT gene are ligated at each terminator side (see, Fig. 1). A vector derived from p35S-ACE1-CR and pKXS-MRE4/35S(-46)-sGFP was referred to as vector (a), a vector derived from p35-ACE1DBD/VP16AD-CR and pKXS-MRE4/35S(-46)-sGFP was referred to as vector (b), a vector derived from p35S-ACE1/VP16AD-CR and pKXS-MRE4/35S (-46) -sGFP was referred to as vector (c), a derived from p35S-ACE1/VP16AD-CR and pKXS-MRE4/35S (-90m) -sGFP was referred to as vector (d), and a vector derived from p35S-ACE1/VP16AD-CR and pKXS-MRE4/35S(- 90m)-FT was referred to as vector (e).
HEK-1F : 5'-agcttgaattcgtcgacggtacctaggacgagctc-3' (SEQ ID NO:30)
HEK-1RC : 5'-aattgagctcgtcctaggtaccgtcgacgaattca-3' (SEQ ID NO:31)

### Example 2 (Analysis of GFP accumulation amount in recombinant tobacco cultured cells)

### (1) Preparation and selection of recombinant tobacco cultured cells

Each of vectors (a), (b), (c) and (d) produced in Example 1 was introduced into tobacco cultured cells (BY-2) by using gold particles of 1.0 µm in diameter coated with each of these vectors according to a particle gun method (Morikawa Hiromichi et al, 1992, Plant Cell Engineering, Vol.4 No.1 p.47-52, Shujunsha Co., Ltd.). The DNA amount per 1.0 mg of gold particles was adjusted to 0.1 µg. On the 3rd to 5th days after gene introduction operation, the tobacco cell suspension cultures subjected to gene introduction were spread on a modified MS agar medium (MS inorganic salts, 3% sucrose, 1 µM 2,4-D, 1 mg/L thiamin HCl, 100 mg/L myo-inositol, 200 mg/L KH₂ PO₄, 0.8% agar) containing 30 mg/L of kanamycin. After culturing for one month, cell mass exhibiting resistance to 30 mg/L kanamycin was selected and the selected cell masses were again cultured for 4 to 8 weeks while they are transferred to a new agar medium every one or two weeks. The condition for culture was at 23 to 25°C in a dark place.

### (2) Analysis of GFP accumulation amount using a fluorescent plate reader

The obtained cell mass was transplanted onto modified MS agar medium (MS inorganic salts, 3% sucrose, 1 µM 2, 4-D, 1 mg/L thiamin HCl, 100 mg/L myo-inositol, 200 mg/L KH₂PO₄, 0.8% agar) containing 100 µM CuSO₄ as an inducible expression treatment group to carry out a treatment for induced expression of a target heterologous gene by copper ion (hereinafter, sometimes, referred to as the inducible expression treatment). Additionally, as a non-inducible expression treatment group, a modified MS agar medium not containing CuSO₄ was used.

Three days after the inducible expression treatment, the cell mass was examined under a fluorescent microscope (Nikon) to determine the fluorescent emission state of GFP.

The cell mass confirmed with the increased fluorescent emission of GFP was frozen in liquid nitrogen. Then, to the frozen cells, glass beads (0.25 to 0.5 mm) and an extraction buffer (1×PBS (-), 5 mM DTT, 1 mM PMSF, 0.1% protease inhibitor cocktail) were added, followed by disruption treatment using a disruption device, "Mixer Mill" (QIAGEN). The resulting disruption product was centrifuged by a table centrifugal machine at 15000 rpm, 4°C for five minutes to obtain the supernatant, which was used as a protein extraction solution. Subsequently, the protein extraction solution was diluted appropriately with 1×PBS (-), and the fluorescence intensity of GFP in the obtained diluted solution was determined by using a multi label counter "Wallac 1420 ARVOMX" (Perkin Elmer). Meanwhile, as a standard sample, a dilution obtained by diluting recombinant GFP (Cosmo Bio) with 1×PBS (- ) was used. From the measured fluorescence intensity, GFP conversion amount per unit amount of soluble proteins was obtained. Further, by subtracting the corresponding value for the control wild-type cell mass as background value, the GFP accumulation amount was calculated. In addition, the protein concentration in the protein extraction solution was quantified according to the Bradford method.

In Table 1, a quantification result of GFP accumulated in the recombinant tobacco cultured cells, in which sGFP gene expression vector that can be induced by copper ions is introduced, is summarized. Induction ratio indicates the ratio of accumulated amount of GFP in the inducible expression treatment group compared to the accumulated amount of GFP in the non-inducible expression treatment group.

For the recombinant tobacco cultured cells into which the vector (a) has been introduced, the accumulated amount of GFP in the inducible expression treatment group was two times higher than that of GFP in the non-inducible expression treatment group (hereinafter, sometimes also referred to as "induction ratio for the recombinant tobacco cultured cells into which the vector (a) has been introduced). For the recombinant tobacco cultured cells into which the vector (b) has been introduced in which ACE1AD of the ACE1 transcription factor has been replaced by VP16AD, the accumulated amount of GFP in the inducible expression treatment group was four times higher than that of GFP in the non-inducible expression treatment group (hereinafter, sometimes also referred to as "induction ratio for the recombinant tobacco cultured cells into which the vector (b) has been introduced), and it was recognized that it was improved two times compared to the induction ratio of the recombinant tobacco cultured cells into which the vector (a) has been introduced.

Meanwhile, for the recombinant tobacco cultured cells into which the vector (c) has been introduced in which VP16AD has been added to the ACE1 transcription factor, the accumulated amount of GFP in the inducible expression treatment group was eighteen times higher than that of GFP in the non-inducible expression treatment group (hereinafter, sometimes also referred to as "induction ratio for the recombinant tobacco cultured cells into which the vector (c) has been introduced), and it was recognized that it was improved nine times compared to the induction ratio of the recombinant tobacco cultured cells into which the vector (a) was introduced.

Still further, for the recombinant tobacco cultured cells into which the vector (d) has been introduced in which 35S(-90m) sequence having a mutation at as-1 site is used, the accumulated amount of GFP in the inducible expression treatment group was eighteen times higher than that of GFP in the non-inducible expression treatment group (hereinafter, sometimes also referred to as "induction ratio for the recombinant tobacco cultured cells into which the vector (d) has been introduced), and it was recognized that it was improved nine times compared to the induction ratio of the recombinant tobacco cultured cells into which the vector (a) has been introduced.

**Table 1**

| Vector | GFP (ng/µg protein) | | Induction ratio | Improvement ratio |
|---|---|---|---|---|
| | Non-inducible expression treatment gruop | Inducible expression treatment group | | |
| (a) | 0.5 | 1.0 | 2 | 1 |
| (b) | 1.1 | 4.2 | 4 | 2 |
| (c) | 0.8 | 14.2 | 18 | 9 |
| (d) | 0.6 | 10.8 | 18 | 9 |

### Example 3 (Flowering induction in recombinant Arabidopsis thaliana)

### (1) Preparation and selection of recombinant Arabidopsis thaliana

The vector (e) prepared in Example 1 was introduced into Agrobacterium (*Agrobacterium timefaciens* strain C58C1). The resulting Agrobacterium was cultured in a LB agar medium (0.5% yeast extract, 1.0% bactotrypton, 0.5% saline, 1% agar) containing 50 mg/L kanamycin, 100 mg/L ampicillin, and 100 mg/L rifampicin, and a drug-resistant colony was selected to obtain recombinant Agrobacterium. Arabidopsis (Arabidopsis thaliana ecotype Columbia) was infected by the obtained recombinant Acrobacterium according to the method described in Model Plant Laboratory Manual (edited by Iwabuchi Masaki et al, 2000, Springer-Verlag Tokyo Co., Ltd., ISBN 4-431-70881-2 C3045) to introduce genes. After T₁ seeds collected from the Arabidopsis subjected to gene introduction, the seeds were plated and grown on a modified MS agar medium (MS inorganic salts, B5 vitamin, 1% sucrose, 0.8% agar) containing 20 mg/L of Benlate, 200 mg/L of Claforan, and 25 mg/L kanamycin to select a plant individual resistant to kanamycin. The selected plant individual was transplanted to a pot in which culture soil was previously placed, and grown in an artificial weather chamber to obtain T₂ seeds. After the obtained T₂ seeds were plated and grown on a modified MS agar medium (MS inorganic salts, B5 vitamin, 2% sucrose, 0-8% agar) containing 25 mg/L kanamycin, a plant line which produces the kanamycin-resistant individual plant in 3:1 ratio at 5% significance level according to chi-square test was selected. Meanwhile, the condition for cultivating the plant included 23 hours of light period and 1 hour of dark period at 23 to 2,5°C.

### (2) Flowering induction in recombinant Arabidopsis thaliana

With respect to the selected line, T₂ seeds were plated on a modified MS agar medium (MS inorganic salts, B5 vitamin, 2% sucrose, 0.8% agar). After incubating the individual plant for 11 days under the condition including 23 hours of light period and 1 hour of dark period at 23 to 25°C, the resulting plant was transplanted to a modified MS agar medium (MS inorganic salts, B5 vitamin, 2% sucrose, 0.8% agar) containing 50 µM CuSO₄, to carry out a treatment for inducible expression of a heterologous gene of interest by copper ions (hereinafter, sometimes referred to as inducible expression treatment). In addition, as a non-inducible expression treatment group, a modified MS agar medium not containing CuSO₄ was used.

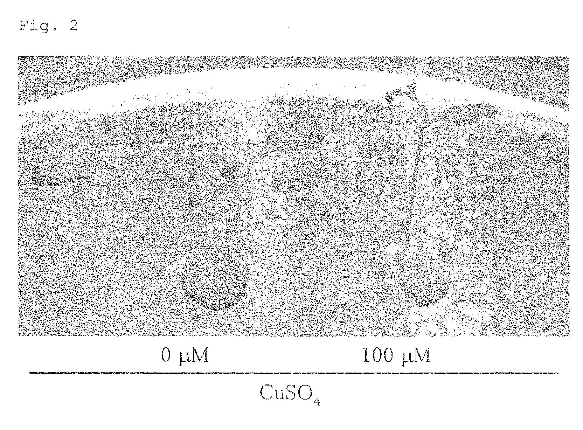

Six hours after the inducible expression treatment, the individual plant was transferred to the modified MS agar medium (MS inorganic salts, B5 vitamin, 2% sucrose, 0.8% agar) not containing CuSO₄. Five days after the inducible expression treatment, observation of the individual plant was carried out. As a result, it was found that flowering was induced earlier in the inducible expression treatment group compared to non-inducible expression treatment group (see, Fig. 2).

According to the present invention, a method for control of flowering time in a transgenic plant in a copper-inducible manner is provided, wherein a heterologous gene which controls flowering time has been introduced to the transgenic plant.

## Claims

1. A method for controlling flowering time of a plant, which comprises bringing a plant into contact with copper ions, wherein the plant has been transformed with the following DNA constructs (i) and (ii):
(i) a first DNA construct comprising a nucleotide sequence encoding a chimeric transcription factor, wherein the chimeric transcription factor comprises the following a) and b) as operably linked elements:
a) a DNA-binding domain and a transcriptional activation domain of a first transcription factor that is capable of being activated by copper ion, and
b) a transcriptional activation domain of at least one second transcription factor that is different from the first transcription factor; and
(ii) a second DNA construct encoding a heterologous gene which controls flowering time of a plant, wherein the gene is under the control of a heterologous promoter capable of being stimulated by the chimeric transcription factor of (i) in the presence of copper ion.

2. The method according to claim 1, wherein the first DNA construct further comprises a terminator which is capable of functioning in plant cells and is operably linked to the nucleotide sequence encoding the chimeric transcription factor, and wherein the second DNA construct further comprises a terminator which is capable of functioning in plant cells and is operably linked to the coding sequence in the heterologous gene.

3. The method according to claim 1 or 2, wherein the first transcription factor is selected from the group consisting of:
(1) a eukaryote transcription factor which is capable of being activated by copper ion,
(2) a yeast transcription factor which is capable of being ctivated by copper ion, and
(3) a transcription factor derived from the yeast ACE1 which is capable of being activated by copper ion.

4. The method according to any of claims 1 to 3, wherein the second transcription factor is selected from the group consisting of:
(1) a transcription factor of a virus,
(2) a transcription factor of a virus belonging to Simplex virus genus,
(3) a transcription factor of Herpes simplex virus, and
(4) the VP16 transcription factor of Herpes simplex virus.

5. The method according to any of claims 1 to 4, wherein the heterologous gene which controls flowering time of a plant is selected from the group consisting of:
(1) a plant gene controlling flowering time,
(2) an angiosperm gene controlling flowering time,
(3) a dicotyledonous gene controlling flowering time,
(4) a Cruciferous gene controlling flowering time,
(5) an *Arabidopsis thaliana* gene controlling flowering time, and
(6) a flowering-time controlling gene derived from the *Arabidopsis thaliana* FT gene.

6. The method according to any of claims 1 to 5, wherein the first DNA construct further comprises a promoter which is capable of functioning in plant cells and is operably linked to the nucleotide sequence encoding the chimeric transcription factor.

## Patentansprüche

1. Verfahren zur Kontrolle der Blütezeit einer Pflanze, das das Inkontaktbringen der Pflanze mit Kupferionen umfasst, wobei die Pflanze mit den folgenden DNA-Konstrukten (i) und (ii) transformiert wurde:
(i) einem ersten DNA-Konstrukt, das eine Nucleotidsequenz umfasst, die einen chimären Transkriptionsfaktor codiert, wobei der chimäre Transkriptionsfaktor die folgenden a) und b) als funktionell verknüpfte Elemente umfasst:
a) eine DNA-bindende Domäne und eine transkriptionelle Aktivierungsdomäne eines ersten Transkriptionsfaktors, der sich durch Kupferionen aktivieren lässt, und
b) eine transkriptionelle Aktivierungsdomäne von mindestens einem zweiten Transkriptionsfaktor, der sich von dem ersten Transkriptionsfaktor unterscheidet; und
(ii) einem zweiten DNA-Konstrukt, das ein heterologes Gen codiert, das die Blütezeit einer Pflanze kontrolliert, wobei das Gen unter der Kontrolle eines heterologen Promotors ist, der sich durch den chimären Transkriptionsfaktor von (i) in Anwesenheit von Kupferionen stimulieren lässt.

2. Verfahren nach Anspruch 1, wobei das erste DNA-Konstrukt des Weiteren einen Terminator umfasst, der fähig ist, in Pflanzenzellen zu fungieren, und der mit der Nucleotidsequenz funktionell verknüpft ist, die den chimären Transkriptionsfaktor codiert, und wobei das zweite DNA-Konstrukt des Weiteren einen Terminator umfasst, der fähig ist, in Pflanzenzellen zu fungieren, und der mit der codierenden Sequenz im heterologen Gen funktionell verknüpft ist.

3. Verfahren nach Anspruch 1 oder 2, wobei der erste Transkriptionsfaktor ausgewählt ist aus der Gruppe bestehend aus:
(1) einem eukaryotischen Transkriptionsfaktor, der sich durch Kupferionen aktivieren lässt,
(2) einem Hefe-Transkriptionsfaktor, der sich durch Kupferionen aktivieren lässt, und
(3) einem aus dem ACE1 der Hefe stammenden Transkriptionsfaktor, der sich durch Kupferionen aktivieren lässt.

4. Verfahren nach einem der Ansprüche 1 bis 3, wobei der zweite Transkriptionsfaktor ausgewählt ist aus der Gruppe bestehend aus:
(1) einem Transkriptionsfaktor eines Virus,
(2) einem Transkriptionsfaktor eines Virus, der zu der Gattung Simplexvirus gehört,
(3) einem Transkriptionsfaktor des Herpes-simplex-Virus,
(4) dem VP16-Transkriptionsfaktor des Herpes-simplex-Virus.

5. Verfahren nach einem der Ansprüche 1 bis 4, wobei das heterologe Gen, das die Blütezeit einer Pflanze kontrolliert, ausgewählt ist aus der Gruppe bestehend aus:
(1) einem Pflanzengen, das die Blütezeit kontrolliert,
(2) einem Angiospermen-Gen, das die Blütezeit kontrolliert,
(3) einem Dikotylen-Gen, das die Blütezeit kontrolliert,
(4) einem Kreuzblütler-Gen, das die Blütezeit kontrolliert,
(5) einem *Arabidopsis thaliana*-Gen, das die Blütezeit kontrolliert, und
(6) einem die Blütezeit kontrollierendem Gen, das aus dem *Arabidopsis thaliana-FT-Gen* stammt.

6. Verfahren nach einem der Ansprüche 1 bis 5, wobei das erste DNA-Konstrukt des Weiteren einen Promotor umfasst, der fähig ist, in Pflanzenzellen zu fungieren, und mit der Nucleotidsequenz, die den chimären Transkriptionsfaktor codiert, funktionell verknüpft ist.

## Revendications

1. Procédé de contrôle du temps de floraison d'une plante, qui comprend la mise en contact d'une plante avec des ions cuivre, où la plante a été transformée avec les produits de recombinaison d'ADN (i) et (ii) suivants :
(i) un premier produit de recombinaison d'ADN comprenant une séquence de nucléotides codant pour un facteur de transcription chimérique, où le facteur de transcription chimérique comprend a) et b) ci-dessous en tant qu'éléments en liaison fonctionnelle :
a) un domaine de liaison à l'ADN et un domaine d'activation de la transcription d'un premier facteur de transcription qui est capable d'être activé par un ion cuivre, et
b) un domaine d'activation de la transcription d'au moins un second facteur de transcription qui est différent du premier facteur de transcription ; et
(ii) un second produit de recombinaison d'ADN codant pour un gène hétérologue qui contrôle le temps de floraison d'une plante, où le gène est sous le contrôle d'un promoteur hétérologue capable d'être stimulé par le facteur de transcription chimérique de (i) en présence d'un ion cuivre.

2. Procédé selon la revendication 1, dans lequel le premier produit de recombinaison d'ADN comprend en outre un terminateur qui est capable de fonctionner dans des cellules végétales et est en liaison fonctionnelle avec la séquence de nucléotides codant pour le facteur de transcription chimérique, et où le second produit de recombinaison d'ADN comprend en outre un terminateur qui est capable de fonctionner dans des cellules végétales et est en liaison fonctionnelle avec la séquence codante dans le gène hétérologue.

3. Procédé selon la revendication 1 ou 2, dans lequel le premier facteur de transcription est sélectionné dans le groupe consistant en :
(1) un facteur de transcription eucaryote qui est capable d'être activé par un ion cuivre,
(2) un facteur de transcription de levure qui est capable d'être activé par un ion cuivre, et
(3) un facteur de transcription dérivé de ACE1 de la levure qui est capable d'être activé par un ion cuivre.

4. Procédé selon l'une quelconque des revendications 1 à 3, dans lequel le second facteur de transcription est sélectionné dans le groupe consistant en :
(1) un facteur de transcription d'un virus,
(2) un facteur de transcription d'un virus appartenant au genre virus Simplex,
(3) un facteur de transcription du virus Herpès simplex, et
(4) le facteur de transcription VP16 du virus Herpès simplex.

5. Procédé selon l'une quelconque des revendications 1 à 4, dans lequel le gène hétérologue qui contrôle le temps de floraison d'une plante est sélectionné dans le groupe consistant en :
(1) un gène végétal contrôlant le temps de floraison,
(2) un gène d'angiosperme contrôlant le temps de floraison,
(3) un gène de dicotylédone contrôlant le temps de floraison,
(4) un gène de crucifère contrôlant le temps de floraison,
(5) un gène d'*Arabidopsis thaliana* contrôlant le temps de floraison, et
(6) un gène contrôlant le temps de floraison dérivé du gène FT d'*Arabidopsis thaliana.*

6. Procédé selon l'une quelconque des revendications 1 à 5, dans lequel le premier produit de recombinaison d'ADN comprend en outre un promoteur qui est capable de fonctionner dans des cellules végétales et est en liaison fonctionnelle avec la séquence de nucléotides codant pour le facteur de transcription chimérique.


### SEQUENCE LISTING

<110> SUMITOMO CHEMICAL COMPANY, LIMITED
<120> METHOD FOR CONTROLLING FLOWERING TIME OF PLANT
<130> R2530 EP S3
<150> JP2008-225711
   <151> 2008-09-03
<160> 31
<210> 1
   <211> 26
   <212> DNA
   <213> Artificial Sequence
<220>
   <221> Source
   <223> /note = "Description of Artificial Sequence: Designed oligonucleotide primer for PCR"
<400> 1
   atggatccat ggtcgtaatt aacggg 26
<210> 2
   <211> 29
   <212> DNA
   <213> Artificial Sequence
<220>
   <221> Source
   <223> /note = "Description of Artificial Sequence: Designed oligonucleotide primer for PCR"
<400> 2
   tggagctctt attgtgaatg tgagttatg 29
<210> 3
   <211> 28
   <212> DNA
   <213> Artificial Sequence
<220>
   <221> Source
   <223> /note = "Description of Artificial Sequence: Designed oligonucleotide primer for PCR"
<400> 3
   aactcgagtt gtgaatgtga gttatgcg 28
<210> 4
   <211> 21
   <212> DNA
   <213> Artificial Sequence
<220>
   <221> Source
   <223> /note = "Description of Artificial Sequence: Designed oligonucleotide primer for PCR"
<400> 4
   acggctccac cgaccgacgt c 21
<210> 5
   <211> 23
   <212> DNA
   <213> Artificial Sequence
<220>
   <221> Source
   <223> /note = "Description of Artificial Sequence: Designed oligonucleotide primer for PCR"
<400> 5
   ctacccaccg tactcgtcaa ttc 23
<210> 6
   <211> 21
   <212> DNA
   <213> Artificial Sequence
<220>
   <221> Source
   <223> /note = "Description of Artificial Sequence: Designed oligonucleotide primer for PCR"
<400> 6
   ggacgaactc cacttagacg g 21
<210> 7
   <211> 21
   <212> DNA
   <213> Artificial Sequence
<220>
   <221> Source
   <223> /note = "Description of Artificial Sequence: Designed oligonucleotide primer for PCR"
<400> 7
   ccgtctaagt ggagttcgtc c 21
<210> 8
   <211> 31
   <212> DNA
   <213> Artificial Sequence
<220>
   <221> Source
   <223> /note = "Description of Artificial Sequence: Designed oligonucleotide primer for PCR"
<400> 8
   tartcgagtc aacggctcca ccgaccgacg t 31
<210> 9
   <211> 35
   <212> DNA
   <213> Artificial Sequence
<220>
   <221> Source
   <223> /note = "Description of Artificial Sequence: Designed oligonucleotide primer for PCR"
<400> 9
   aagagctctt acccaccgta ctcgtcaatt ccaag 35
<210> 10
   <211> 32
   <212> DNA
   <213> Artificial Sequence
<220>
   <221> Source
   <223> /rote = "Description of Artificial Sequence: Designed oligonucleotide primer for PCR"
<400> 10
   taagatctat caacggctcc accgaccgac gt 32
<210> 11
   <211> 15
   <212> DNA
   <213> Artificial Sequence
<220>
   <221> Source
   <223> /note = "Description of Artificial Sequence: Designed oligonucleotide for adapter"
<400> 11
   ggccgcgagc tcagt 15
<210> 12
   <211> 12
   <212> DNA
   <213> Artificial Sequence
<220>
   <221> Source
   <223> /note = "Description of Artificial Sequence: Designed oligonucleotide for adapter"
<900> 12
   gactgagctc gc 12
<210> 13
   <211> 52
   <212> DNA
   <213> Artificial Sequence
<220>
   <221> Source
   <223> /note = "Description of Artificial Sequence: Designed oligonucleotide for DNA fragment to be substituted"
<400> 13
   agcttagcga tgcgtctttt ccgctgaacc gttccagcaa aaaagactag at 52
<210> 14
   <211> 48
   <212> DNA
   <213> Artificial Sequence
<220>
   <221> Source
   <223> /note = "Description of Artificial Sequence: Designed oligonucleotide for DNA fragment to be substituted"
<400> 14
   atctagtctt ttttgctgga acggttcagc ggaaaagacg catcgcta 48
<210> 15
   <211> 32
   <212> DNA
   <213> Artificial Sequence
<220>
   <221> Source
   <223> /note = "Description of Artificial Sequence: Designed oligonucleotide primer for PCR"
<400> 15
   tagatatcgc aagacccttc ctctatataa gg 32
<210> 16
   <211> 23
   <212> DNA
   <213> Artificial Sequence
<220>
   <221> Source
   <223> /note = "Description of Artificial Sequence: Designed oligonucleotide primer for PCR"
<400> 16
   atcctctaga gtcccccgtg ttc 23
<210> 17
   <211> 27
   <212> DNA
   <213> Artificial Sequence
<220>
   <221> Source
   <223> /note = "Description of Artificial Sequence: Designed oligonucleotide primer for PCR"
<400> 17
   gctatgacca tgattacgcc aagcttg 27
<210> 18
   <211> 27
   <212> DNA
   <213> Artificial Sequence
<220>
   <221> Source
   <223> /note = "Description of Artificial Sequence: Designed oligonucleotide primer for PCR"
<400> 18
   cattgttata tctccttgga tccgtcg 27
<210> 19
   <211> 26
   <212> DNA
   <213> Artificial Sequence
<220>
   <221> Source
   <223> /note = "Description of Artificial Sequence: Designed oligonucleotide primer for PCR"
<400> 19
   tagatatctc cacgtccata agggac 26
<210> 20
   <211> 27
   <212> DNA
   <213> Artificial Sequence
<220>
   <221> Source
   <223> /note = "Description of Artificial Sequence: Designed oligonucleotide primer for PCR"
<400> 20
   aatctagact gcaggtcgtc ctctcca 27
<210> 21
   <211> 16
   <212> DNA
   <213> Artificial Sequence
<220>
   <221> Source
   <223> /note = "Description of Artificial Sequence: Designed oligonucleotide for adapter"
<400> 21
   ggtacctcga gtcgac 16
<210> 22
   <211> 16
   <212> DNA
   <213> Artificial Sequence
<220>
   <221> Source
   <223> /note = "Description of Artificial Sequence: Designed oligonucleotide for adapter"
<400> 22
   gtcgactcga ggtacc 16
<210> 23
   <211> 34
   <212> DNA
   <213> Artificial Sequence
<220>
   <221> Source
   <223> /note = "Description of Artificial Sequence: Designed oligonucleotide primer for PCR"
<400> 23
   taatctagaa tgtctataaa tataagagac cctc 34
<210> 24
   <211> 28
   <212> DNA
   <213> Artificial Sequence
<220>
   <221> Source
   <223> /note = "Description of Artificial Sequence: Designed oligonucleotide primer for PCR"
<400> 24
   atagagctcc taaagtcttc ttcctccg 28
<210> 25
   <211> 34
   <212> DNA
   <213> Artificial Sequence
<220>
   <221> Source
   <223> /note = "Description of Artificial Sequence: Designed oligonucleotide primer for PCR"
<400> 25
   taaggatcca tgtctataaa tataagagac cctc 34
<210> 26
   <211> 29
   <212> DNA
   <213> Artificial Sequence
<220>
   <221> Source
   <223> /note = "Description of Artificial Sequence: Designed oligonucleotide primer for PCR"
<400> 26
   gaacatctgg atcgaccata accaaagta 29
<210> 27
   <211> 29
   <212> DNA
   <213> Artificial Sequence
<220>
   <221> Source
   <223> /note = "Description of Artificial Sequence: Designed oligonucleotide primer for PCR"
<400> 27
   tactttggtt atggtcgatc cagatgttc 29
<210> 28
   <211> 25
   <212> DNA
   <213> Artificial Sequence
<220>
   <221> Source
   <223> /note = "Description of Artificial Sequence: Designed oligonucleotide primer for PCR"
<400> 28
   gacacgatga atacctgcag tggga 25
<210> 29
   <211> 25
   <212> DNA
   <213> Artificial Sequence
<220>
   <221> Source
   <223> /note = "Description of Artificial Sequence: Designed oligonucleotide primer for PCR"
<400> 29
   tcccactgca ggtattcatc gtgtc 25
<210> 30
   <211> 35
   <212> DNA
   <213> Artificial Sequence
<220>
   <221> Source
   <223> /note = "Description of Artificial Sequence: Designed oligonucleotide for adapter"
<400> 30
   agcttgaatt cgtcgacgg tacctaggac gagctc 35
<210> 31
   <211> 35
   <212> DNA
   <213> Artificial Sequence
<220>
   <221> Source
   <223> /note = "Description of Artificial Sequence: Designed oligonucleotide for adapter"
<400> 31
   aattgagctc gtcctaggta ccgtcgacga attca 35